# EUROPÄISCHE PATENTSCHRIFT

(11) **EP 0 464 867 B1**
(45) Veröffentlichungstag und Bekanntmachung des Hinweises auf die Patenterteilung: **10.05.1995**
(21) Anmeldenummer: 91114412.9
(22) Anmeldetag: 21.11.1986
(51) Int. Cl.: C12N 15/62, C12N 15/15, C12N 15/26, C12N 15/70

(54) **Fusionsproteine aus Interleukin-2 und Hirudin**
Fusion proteins of interleukin-alpha with hirudin
Protéines de fusion de l'interleukine-alpha et l'hirudine

(30) Priorität: 27.11.1985 DE 3541856
(43) Veröffentlichungstag der Anmeldung: 08.01.1992
(62) Teilanmeldung aus: 86116140.4
(73) Patentinhaber: HOECHST AKTIENGESELLSCHAFT, 65926 Frankfurt am Main (DE)
(72) Erfinder: Habermann, Paul, Dr., W-6230 Frankfurt a m Main 80 (DE); Wengenmyer, Friedrich, Dr., W-6238 Hofheim a Ts. (DE)

(56) Entgegenhaltungen:
- EP-A- 0 155 655
- EP-A- 0 158 198
- EP-A- 0 158 564
- EP-A- 0 171 024
- EP-A- 0 211 299

## Beschreibung

Die Erfindung bezieht sich auf Fusionsproteine aus Interleukin-2 und Hirudin.

Bei der gentechnischen Herstellung eukaryotischer Proteine wird in Bakterien häufig nur eine geringe Ausbeute erhatten, insbesondere bei kleinen Proteinen mit einem Molgewicht bis zu etwa 15 000 Dalton, wie Hirudin, deren Strukturen Disulfidbrücken enthalten. Man nimmt an, daß die gebildeten Proteine durch wirtseigene Proteasen rasch abgebaut werden. Man konstruiert deshalb zweckmäßig Genstrukturen, die für Fusionsproteine codieren, wobei der im allgemeinen unerwünschte Anteil des Fusionsproteins ein wirtseigenes Protein ist, das nach der Isolation der Primärproduktes nach an sich bekannten Methoden abgespalten wird.

Es wurde nun überraschenderweise gefunden, daß ein N-terminaler Anteil von Interleukin-2, der im wesentlichen den ersten 100 Aminosäuren entspricht, besonders gut zur Herstellung von Fusionsproteinen geeignet ist. Man erhält also als Primärprodukt ein Fusionsprotein, das völlig oder zum ganz überwiegenden Teil aus eukaryotischen Proteinsequenzen besteht. Überraschenderweise wird dieses Protein offenbar in dem betreffenden Wirtsorganismus nicht als Fremdprotein erkannt und nicht sofort wieder abgebaut. Ein weiterer Vorteil ist, daß die erfindungsgemäßen Fusionsproteine schwer löslich bis unlöslich sind und sich somit einfach, zweckmäßig durch Zentrifugation, von den löslichen Proteinen abtrennen lassen.

Die für Human-Interleukin-2, im folgenden "IL-2", codierende natürliche DNA-Sequenz ist aus der europäischen Patentanmeldung mit der Veröffentlichungsnummer EP-A-1-0 091 539 bekannt. Zweckmäßiger geht man jedoch von einer synthetischen DNA aus, besonders vorteilhaft von der DNA für Human-IL-2, die in der (nicht voveröffentlichten) deutschen Offenlegungsschrift 34 19 995 (entsprechend der unter der Nummer 0 163 249 veröffentlichten europäischen Patentanmeldung) vorgeschlagen wurde. Diese synthetische DNA-Sequenz ist im Anhang wiedergegeben (DNA-Sequenz I). Diese synthetische DNA hat nicht nur den Vorzug, daß sie in der Codon-Wahl auf die Gegebenheiten des am häufigsten verwendeten Wirts, E. coli, abgestimmt ist, sondern sie enthält auch eine Reihe von Schnittstellen für Restriktionsendonucleasen, von denen erfindungsgemäß Gebrauch gemacht werden kann.

Man nutzt hierbei die DNA-Sequenz - mehr oder weniger - bis zum Ende und erzeugt so - ein gegebenenfalls modifiziertes - bifunktionelles Protein, das IL-2 Wirkung zusätzlich zur Wirkung des Hirudin zeigt.

Die Erfindung betrifft somit Fusionsproteine der allgemeinen Formel

Met - X - Y - Z (Ia)

oder

Met - Z - Y - X (Ib)

in der X im wesentlichen die Aminosäurefolge von vorzugsweise menschlichem IL-2 bedeutet, Y eine direkte Bindung oder ein Brückenglied aus einer oder mehreren genetisch codierbaren Aminosäuren, und Z Hirudin ist.

Wie sich aus den Formeln Ia und Ib ergibt - und wie es auch schon vorstehend erwähnt wurde - ist es möglich, das Hirudin vor oder nach dem IL-2-Anteil zur Expression zu bringen. Zur Vereinfachung wird im folgenden im wesentlichen die erste Möglichkeit erläutert, die der herkömmlichen Methode zur Herstellung von Fusionsproteinen entspricht. Wenn also im folgenden diese "klassische" Variante beschrieben wird, soll die andere Alternative hierdurch nicht ausgeschlossen werden.

Das Fusionsprotein wird durch Expression in einem geeigneten Expressionssystem in an sich bekannter Weise gewonnen. Hierfür eignen sich alle bekannten Wirts-Vektor-Systeme, also beispielsweise Säugerzellen und Mikroorganismen, beispielsweise Hefen und vorzugsweise Bakterien, insbesondere E. coli.

Die DNA-Sequenz, die für das Hirudin codiert, wird in bekannter Weise in einen Vektor eingebaut, der in dem gewählten Expressionssystem eine gute Expression gewährleistet.

In bakteriellen Wirten wählt man zweckmäßig den Promotor und Operator aus der Gruppe lac, tac, trp, P_{L} oder P_{R} des Phagen λ, hsp, omp oder einen synthetischen Promotor, wie sie beispielsweise in der deutschen Offenlegungsschrift 34 30 683 (Europäische Patentanmeldung mit der Veröffentlichungsnummer 0 173 149) vorgeschlagen sind. Vorteilhaft ist die tac PromotorOperator-Sequenz, die inzwischen handelsüblich ist (z.B. Expressionsvektor pKK223-3, Pharmacia, "Molecular Biologicals, Chemicals and Equipment for Molecular Biology", 1984, S. 63).

Bei der Expression des erfindungsgemäßen Fusionsproteins kann es sich als zweckmäßig erweisen, einzelne Tripletts der ersten Aminosäuren nach dem ATG-Start-Codon zu verändern, um eine eventuelle Basenpaarung auf der Ebene der mRNA zu verhindern. Solche Veränderungen, ebenso wie Veränderungen, Deletionen oder Additionen einzelner Aminosäuren im IL-2-Proteinanteil, sind dem Fachmann geläufig und ebenfalls Gegenstand der Erfindung.

In den folgenden Beispielen und in den Figuren wird die Erfindung näher erläutert. Hierbei beziehen sich
Figur 1 und deren Fortsetzung, Figur 1a, auf die Synthese des Plasmids pK360, das für ein Fusionsprotein codiert, welches die Hirudinsequenz aufweist;

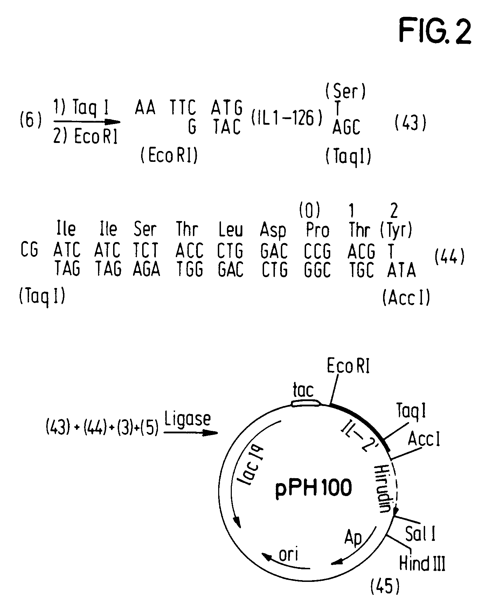

Figur 2 auf die Synthese des Plasmids pPH100, welches für ein Fusionsprotein mit der Aminosäuresequenz des Hirudin codiert.

Die Figuren sind i.a. nicht maßstabgerecht gezeichnet, vor allem bei der Wiedergabe der Polylinker wurde der Maßstab "gedehnt".

### Beispiel 1

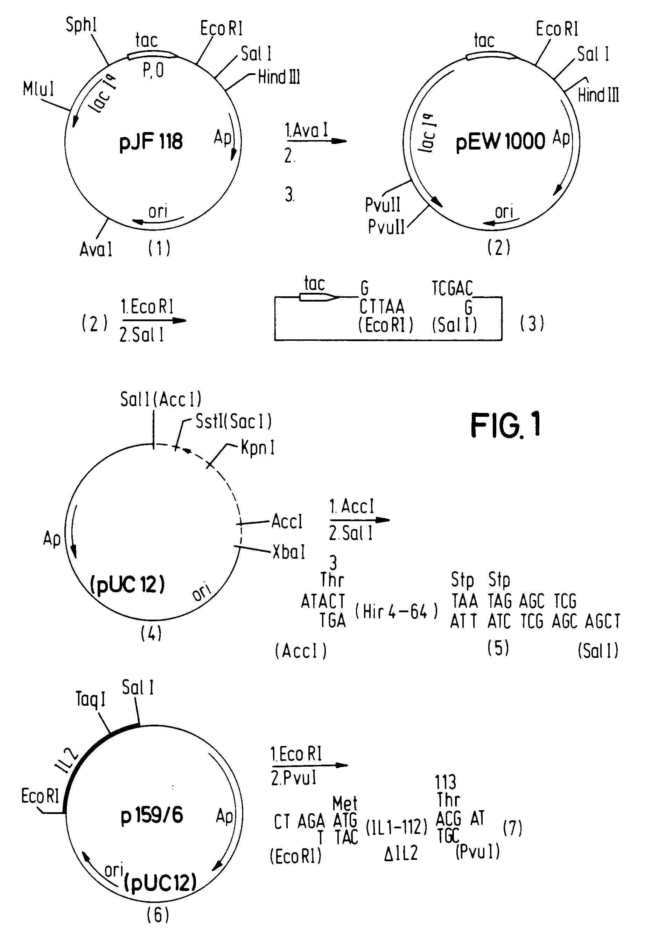

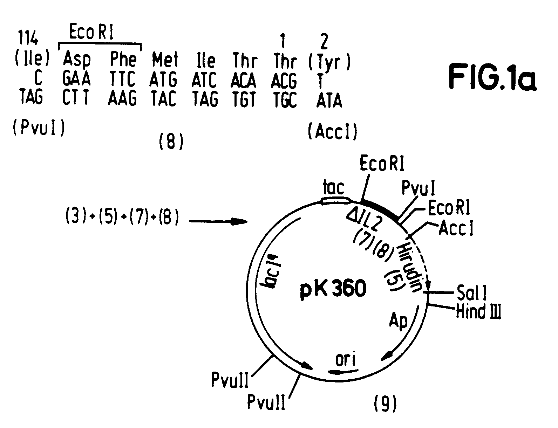

Durch Insertion des lac-Repressors (P.J. Farabaugh, Nature 274 (1978) 765-769) in das Plasmid pKK 177-3 (Amann et al., Gene 25 (1993) 167) erhält man das Plasmid pJF118 (1) (Fig. 1; vgl. deutsche Patentanmeldung P 35 26 995.2, Beispiel 6, Fig. 6). Dieses wird an der singulären Restriktionsstelle für Ava I geöffnet und in an sich bekannter Weise durch Exonuclease-Behandlung um etwa 1000 bp verkleinert. Nach Ligierung wird das Plasmid pEW 1000(2), (Figur 1) erhalten, in dem das lac-Repressorgen vollständig erhalten ist, das aber auf Grund der Verkleinerung in deutlich höherer Kopienzahl als das Ausgangsplasmid vorliegt.

Anstelle des Plasmids pKK177-3 kann man auch von dem vorstehend erwähnten handelsüblichen Plasmid pKK223-3 ausgehen, den lac-Repressor einbauen und das erhaltene Produkt analog verkürzen.

Das Plasmid pEW 1000 (2) wird mit den Restriktionsenzymen EcoR I und Sal I geöffnet (3).

Das für Hirudin codierende Plasmid (4), hergestellt gemäß deutscher Offenlegungsschrift 34 29 430 (europäische Patentanmeldung mit der Veröffentlichungsnummer 0 171 024), Beispiel 4 (Figur 3), wird mit den Restriktionsenzymen Acc I und Sal I geöffnet und das kleine Fragment (5), das zum größten Teil die Hirudin-Sequenz enthält, isoliert.

Das Plasmid p159/6(6), hergestellt gemäß deutscher Offenlegungsschrift 34 19 995 (europäische Patentanmeldung mit der Veröffentlichungsnummer 0 163 249), Beispiel 4 (Figur 5), wird mit den Restriktionsenzymen Eco RI und Pvu I geöffnet und das kleine Fragment (7) isoliert, das den größten Teil der IL2-Sequenz enthält. Diese Teilsequenz und im folgenden auch andere verkürzte IL-2 Sequenzen sind in den Figuren mit "ΔIL2" bezeichnet.

Anschließend werden die Sequenzen (3), (5), (7) sowie die synthetische DNA-Sequenz (8; Figur 1a) mit T4-Ligase behandelt. Man erhält das Plasmid pK360 (9).

Kompetente E. coli-Zellen werden mit dem Ligationsprodukt transformiert und auf NA-Platten, die 25 »g/ml Ampicillin enthalten, ausplattiert. Die Plasmid-DNA der Klone wird mittels Restriktions- und Sequenzanalyse charakterisiert.

Eine Übernachtkultur aus E. coli-Zellen, die das Plasmid (9) enthalten, wird mit LB-Medium (J. H. Miller, Experiments in Molecular Genetics, Cold Spring Harbor Laboratory, 1972), das 50 »g/ml Ampicillin enthält, im Verhältnis von etwa 1:100 verdünnt und das Wachstum über OD-Messung verfolgt. Bei OD = 0,5 wird die Schüttelkultur auf 1 mM Isopropyl-β-galactopyranosid (IPTG) eingestellt und die Bakterien nach 150 bis 180 Minuten abzentrifugiert. Die Bakterien werden 5 Minuten in einer Puffermischung (7M Harnstoff, 0,1% SDS, 0,1 M Natriumphosphat, pH 7,0) gekocht und Proben auf eine SDS-Gelelektrophoreseplatte aufgetragen. Nach Elektrophorese wird aus Bakterien, die das Plasmid (9) enthalten, eine Proteinbande erhalten, die der Größe des erwarteten Fusionsproteins entspricht. Nach Aufschluß der Bakterien (French Press; ®Dyno-Mühle) und Zentrifugation befindet sich das Fusionsprotein im Niederschlag, so daß mit dem Überstand bereits erhebliche Mengen der übrigen Proteine abgetrennt werden können. Nach Isolierung des Fusionsproteins wird durch Bromcyan-Spaltung das erwartete Hirudin-Peptid freigesetzt. Dieses wird nach Isolierung durch Protein-Sequenzanalyse charakterisiert.

Die angegebenen Induktionsbedingungen gelten für Schüttelkulturen; bei größeren Fermentationen sind entsprechend veränderte OD-Werte und gegebenenfalls leicht variierte IPTG-Konzentrationen zweckmäßig.

### Beispiel 2

Das Plasmid (6) (Figur 1) wird mit den Restriktionsenzymen Taq I und Eco RI geöffnet und das kleine Fragment (43) isoliert. Dieses Fragment wird mit der synthetisierten DNA-Sequenz (44) und den Segmenten (3) und (5) zum Plasmid pPH 100(45) ligiert. Dieses Plasmid codiert für ein Fusionsprotein, bei dem auf die ersten 132 Aminosäuren des IL-2 das Brückenglied Asp-Pro und hierauf die Aminosäurefolge von Hirudin folgt. Die proteolytische Spaltung liefert somit ein modifiziertes, biologisch aktives IL-2', in dem in Position 133 an Stelle von Thr Asp enthalten ist, und ein Hirudin-Derivat, das N-terminal Pro vor der Aminosäuresequenz des Naturprodukts enthält. Auch dieses Produkt ist biologisch aktiv und im Vergleich zu dem Naturprodukt stabiler gegen den Angriff von Proteasen.

Das IL-2'-Hirudin-Fusionsprotein zeigt ebenfalls biologische Aktivität:
Im Zellproliferationstest mit einer IL-2-abhängigen Zellinie (CTLL2) wurde biologische Aktivität gefunden.

Nach Denaturierung in 6 M Guanidiniumhydrochlorid-Lösung und anschließende Renaturierung in Pufferlösung (10 mM Tris-HCl, pH 8,5, 1 mM EDTA) wurde weiterhin hohe IL-2-Aktivität gefunden. Außerdem wurde die Gerinnungszeit von säurebehandeltem, mit Thrombin versetztem Blut nach Zugabe des Fusionsproteins verlängert.

Man erhält somit ein bifunktionelles Fusionsprotein.

## Patentansprüche (Patentansprüche für folgende(n) Vertragsstaat(en): BE, CH, DE, FR, GB, IT, LI, LU, NL, SE)

1. Fusionsproteine aus Human-Interleukin 2 (IL-2) und Hirudin, die sowohl IL-2-Aktivität als auch Hirudin-Aktivität zeigen.

2. Plasmid pPH 100 gemäß Fig. 2.

## Patentansprüche (Patentansprüche für folgende(n) Vertragsstaat(en): AT, ES)

1. Verfahren zur Herstellung eines Fusionsproteins, dadurch gekennzeichnet, daß man in einer Wirtszelle ein Gen exprimiert, das für einen C- oder N-terminalen Anteil, der im wesentlichen der Aminosäurefolge von Interleukin-2 (IL-2) entspricht, sowie für Hirudin codiert.

2. Verfahren nach Anspruch 1, dadurch gekennzeichnet, daß das Gen für ein Fusionsprotein der allgemeinen Formel
Met - X - Y - Z (Ia)
oder
Met - Z - Y - X (Ib)
codiert, in der X im wesentlichen die Aminosäurefolge des menschlichen Interleukin-2 bedeutet, Y eine direkte Bindung oder ein Brückenglied aus genetisch codierbaren Aminosäuren bedeutet und Z die Aminosäurefolge des Hirudinderivats ist.

3. Verfahren nach Anspruch 2, dadurch gekennzeichnet, daß Y, benachbart zu Z, Met, Cys, Trp, Arg oder Lys enthält oder aus diesen Aminosäuren besteht.

4. Verfahren nach Anspruch 2, dadurch gekennzeichnet, daß Y, benachbart zu Z, die Aminosäuresequenz
Asp - Pro,
enthält oder aus dieser Sequenz besteht.

5. Verfahren nach einem oder mehreren der vorhergehenden Ansprüche, dadurch gekennzeichnet, daß das Fusionsprotein durch Zentrifugation von den löslichen Proteinen abgetrennt wird.

6. Verfahren nach einem oder mehreren der vorhergehenden Ansprüche, dadurch gekennzeichnet, daß die Wirtszelle ein Bakterium ist.

7. Verfahren nach Anspruch 7, dadurch gekennzeichnet, daß die Wirtszelle E. coli ist.

## Patentansprüche (Patentansprüche für folgende(n) Vertragsstaat(en): GR)

1. Verfahren zur Herstellung eines Fusionsproteins, dadurch gekennzeichnet, daß man in einer Wirtszelle ein Gen exprimiert, das für einen C- oder N-terminalen Anteil, der im wesentlichen der Aminosäurefolge von Interleukin-2 (IL-2) entspricht, sowie für Hirudin codiert.

2. Verfahren nach Anspruch 1, dadurch gekennzeichnet, daß das Gen für ein Fusionsprotein der allgemeinen Formel
Met - X - Y - Z (Ia)
oder
Met - Z - Y - X (Ib)
codiert, in der X im wesentlichen die Aminosäurefolge des menschlichen Interleukin-2 bedeutet, Y eine direkte Bindung oder ein Brückenglied aus genetisch codierbaren Aminosäuren bedeutet und Z die Aminosäurefolge des Hirudinderivats ist.

3. Verfahren nach Anspruch 2, dadurch gekennzeichnet, daß Y, benachbart zu Z, Met, Cys, Trp, Arg oder Lys enthält oder aus diesen Aminosäuren besteht.

4. Verfahren nach Anspruch 2, dadurch gekennzeichnet, daß Y, benachbart zu Z, die Aminosäuresequenz
Asp - Pro,
enthält oder aus dieser Sequenz besteht.

5. Verfahren nach einem oder mehreren der vorhergehenden Ansprüche, dadurch gekennzeichnet, daß das Fusionsprotein durch Zentrifugation von den löslichen Proteinen abgetrennt wird.

6. Verfahren nach einem oder mehreren der vorhergehenden Ansprüche, dadurch gekennzeichnet, daß die Wirtszelle ein Bakterium ist.

7. Verfahren nach Anspruch 7, dadurch gekennzeichnet, daß die Wirtszelle E. coli ist.

8. Plasmid pPH 100 gemäß Fig. 2.

## Claims (Claims for the following Contracting State(s): BE, CH, DE, FR, GB, IT, LI, LU, NL, SE)

1. A fusion protein which is composed of human interleukin-2 (IL-2) and hirudin and which exhibits both IL-2 activity and hirudin activity.

2. A plasmid pPH 100 according to Fig. 2.

## Claims (Claims for the following Contracting State(s): AT, ES)

1. A process for the preparation of a fusion protein, which comprises expression in a host cell of a gene which codes for a C- or N-terminal section which essentially corresponds to the amino acid sequence and codes for hirudin of interleukin-2 (IL-2).

2. The process as claimed in claim 1, wherein the gene codes for a fusion protein of the formula
Met - X - Y - Z (Ia)
or
Met - Z - Y - X (Ib)
in which X essentially denotes the amino acid sequence of human interleukin-2, Y denotes a direct linkage or a bridging element which is composed of genetically encodable amino acids and Z is the amino acid sequence of the hirudin derivative.

3. The process as claimed in claim 2, wherein Y, adjacent to Z, contains Met, Cys, Trp, Arg or Lys, or is composed of these amino acids.

4. The process as claimed in claim 2, wherein Y, adjacent to Z, contains the amino acid sequence
Asp - Pro,
or is composed of this sequence.

5. The process as claimed in one or more of the preceding claims, wherein the fusion protein is removed from the soluble proteins by centrifugation.

6. The process as claimed in one or more of the preceding claims, wherein the host cell is a bacterium.

7. The process as claimed in claim 6, wherein the host cell is E. coli.

## Claims (Claims for the following Contracting State(s): GR)

1. A process for the preparation of a fusion protein, which comprises expression in a host cell of a gene which codes for a C- or N-terminal section which essentially corresponds to the amino acid sequence of interleukin-2 (IL-2) and codes for hirudin.

2. The process as claimed in claim 1, wherein the gene codes for a fusion protein of the formula
Met - X - Y - Z (Ia)
or
Met - Z - Y - X (Ib)
in which X essentially denotes the amino acid sequence of human interleukin-2, Y denotes a direct linkage or a bridging element which is composed of genetically encodable amino acids and Z is the amino acid sequence of the hirudin derivative.

3. The process as claimed in claim 2, wherein Y, adjacent to Z, contains Met, Cys, Trp, Arg or Lys, or is composed of these amino acids.

4. The process as claimed in claim 2, wherein Y, adjacent to Z, contains the amino acid sequence
Asp - Pro,
or is composed of this sequence.

5. The process as claimed in one or more of the preceding claims, wherein the fusion protein is removed from the soluble proteins by centrifugation.

6. The process as claimed in one or more of the preceding claims, wherein the host cell is a bacterium.

7. The process as claimed in claim 6, wherein the host cell is E. coli.

8. A plasmid pPh 100 according to Fig. 2.

## Revendications (Revendications pour l'(les) Etat(s) contractant(s) suivant(s): BE, CH, DE, FR, GB, IT, LI, LU, NL, SE)

1. Protéines de fusion d'interleukine-2 (IL-2) humaine et d'hirudine, qui présentent aussi bien une activité IL-2 qu'une activité d'hirudine.

2. Plasmide pPH100 selon la figure 2.

## Revendications (Revendications pour l'(les) Etat(s) contractant(s) suivant(s): AT, ES)

1. Procédé pour la production d'une protéine de fusion, caractérisé en ce que l'on exprime dans une cellule hôte un gène qui code pour un fragment C- ou N-terminal qui correspond pratiquement à la séquence d'aminoacides de l'interleukine-2 (IL-2), ainsi que pour l'hirudine.

2. Procédé selon la revendication 1, caractérisé en ce que le gène code pour une protéine de fusion de formule générale
Met - X - Y - Z (Ia)
ou
Met - Z - Y - X (Ib)
formules dans lesquelles X représente essentiellement la séquence d'aminoacides de l'interleukine-2 humaine, Y représente une liaison directe ou un chaînon pontant constitué d'aminoacides génétiquement codables, et Z est la séquence d'aminoacides du dérivé d'hirudine.

3. Procédé selon la revendication 2, caractérisé en ce que Y, adjacent à Z, contient Met, Cys, Trp, Arg ou Lys ou consiste en ces aminoacides.

4. Procédé selon la revendication 2, caractérisé en ce que Y, adjacent à Z, contient la séquence d'aminoacides
Asp-Pro
ou consiste en cette séquence.

5. Procédé selon une ou plusieurs des revendications précédentes, caractérisé en ce que la protéine de fusion est séparée par centrifugation d'avec les protéines solubles.

6. Procédé selon une ou plusieurs des revendications précédentes, caractérisé en ce que la cellule hôte est une bactérie.

7. Procédé selon la revendication 6, caractérisé en ce que la cellule hôte est *E. coli.*

## Revendications (Revendications pour l'(les) Etat(s) contractant(s) suivant(s): GR)

1. Procédé pour la production d'une protéine de fusion, caractérisé en ce que l'on exprime dans une cellule hôte un gène qui code pour un fragment C- ou N- terminal qui correspond pratiquement à la séquence d'aminoacides de l'interleukine-2 (IL-2), ainsi que pour l'hirudine.

2. Procédé selon la revendication 1, caractérisé en ce que le gène code pour une protéine de fusion de formule générale
Met - X - Y - Z (Ia)
ou
Met - Z - Y - X (Ib)
formules dans lesquelles X représente essentiellement la séquence d'aminoacides de l'interleukine-2 humaine, Y représente une liaison directe ou un chaînon pontant constitué d'aminoacides génétiquement codables, et Z est la séquence d'aminoacides du dérivé d'hirudine.

3. Procédé selon la revendication 2, caractérisé en ce que Y, adjacent à Z, contient Met, Cys, Trp, Arg ou Lys ou consiste en ces aminoacides.

4. Procédé selon la revendication 2, caractérisé en ce que Y, adjacent à Z, contient la séquence d'aminoacides
Asp-Pro
ou consiste en cette séquence.

5. Procédé selon une ou plusieurs des revendications précédentes, caractérisé en ce que la protéine de fusion est séparée par centrifugation d'avec les protéines solubles.

6. Procédé selon une ou plusieurs des revendications précédentes, caractérisé en ce que la cellule hôte est une bactérie.

7. Procédé selon la revendication 6, caractérisé en ce que la cellule hôte est *E. coli.*

8. Plasmide pPH100 selon la figure 2.
